(19) Europäisches Patentamt
European Patent Office
Office européen des brevets

(11) EP 1 172 091 B1

(12) **FASCICULE DE BREVET EUROPEEN**

(45) Date de publication et mention de la délivrance du brevet:
**07.06.2006 Bulletin 2006/23**

(51) Int Cl.:
***A61K 8/49*** *(2006.01)* ***A61Q 19/04*** *(2006.01)*

(21) Numéro de dépôt: **01401692.7**

(22) Date de dépôt: **26.06.2001**

(54) **Utilisation du 4',5,7-trihydroxyflavylium pour colorer la peau**

Verwendung von 4',5,7-trihydroxyflavylium als Hautfärbemittel

Use of 4',5,7-trihydroxyflavylium for coloring the skin

(84) Etats contractants désignés:
**AT BE CH CY DE DK ES FI FR GB GR IE IT LI LU MC NL PT SE TR**

(30) Priorité: **12.07.2000 FR 0009108**

(43) Date de publication de la demande:
**16.01.2002 Bulletin 2002/03**

(73) Titulaire: **L'OREAL**
**75008 Paris (FR)**

(72) Inventeurs:
- **Candau, Didier**
**91570 Bievres (FR)**
- **Forestier, Serge**
**77140 Claye Souilly (FR)**

(74) Mandataire: **Miszputen, Laurent**
**L'OREAL - D.I.P.I.**
**25-29 Quai Aulagnier**
**92600 Asnières (FR)**

(56) Documents cités:
**FR-A- 2 757 383**

- **DATABASE WPI Week 199929 Derwent Publications Ltd., London, GB; AN 1999-338248 XP002164085 & CN 1 209 992 A (ZHANG)**
- **DATABASE WPI Week 198828 Derwent Publications Ltd., London, GB; AN 1988-195805 XP002164086 & JP 63 135310 A (LION CORP)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

EP 1 172 091 B1

## Description

La présente invention se rapporte à l'utilisation du chlorure de 4',5,7-trihydroxyflavylium, pour conférer à la peau une coloration artificielle proche du bronzage naturel et à leurs utilisations dans le domaine cosmétique susmentionné.

De nos jours, il est important d'avoir bonne mine et une peau bronzée est toujours signe de bonne santé. Cependant, le bronzage naturel n'est pas toujours souhaitable dans la mesure où il nécessite des expositions prolongées aux rayonnements UV, en particulier aux rayonnements UV-A qui provoquent le brunissement de la peau mais en contrepartie sont susceptibles d'induire une altération de celle-ci, notamment dans le cas d'une peau sensible ou d'une peau continuellement exposée au rayonnement solaire. Il est donc souhaitable de trouver une alternative au bronzage naturel qui soit compatible avec les exigences de telles peaux.

La plupart des produits cosmétiques destinés au bronzage artificiel de la peau sont à base de dérivés carbonylés permettant, par interaction avec les acides aminés de la peau, la formation de produits colorés.

A cet effet, on sait que la dihydroxyacétone, ou DHA, est un produit particulièrement intéressant qui est couramment utilisé en cosmétique comme agent de bronzage artificiel de la peau ; appliqué sur cette dernière, notamment sur le visage, il permet d'obtenir un effet de bronzage ou de brunissage d'apparence semblable à celui qui peut résulter d'une exposition prolongée au soleil (bronzage naturel) ou sous une lampe UV.

Un inconvénient de la DHA est la lenteur avec laquelle la coloration se développe : il faut en effet compter plusieurs heures (3 à 5 heures en général) pour que soit révélée la coloration. Il existe donc une demande croissante de produits autobronzants agissant rapidement et conférant une coloration plus proche du bronzage naturel.

Ainsi, on est toujours à la recherche de nouveaux composés et de nouvelles compositions permettant de conférer artificiellement à la peau une coloration proche du bronzage naturel d'une manière simple, efficace, rapide et sans risque.

Les colorants anthocyaniques sont connus depuis longtemps comme colorants alimentaires et pharmaceutiques. Ces anthocyanes sont présents dans la nature sous forme d'hétérosides appelés anthocyanosides et de génines, appelées anthocyanidines. Ces anthocyanes sont des dérivés du phényl-2-benzopyrylium ou flavylium et sont notamment présents dans la plante sous forme de sels. Les anthocyanes sont des composés de teinte rouge, violette ou bleue qui colorent généralement les fleurs, les fruits et parfois les feuilles. La couleur observée dépend à la fois de la structure de la génine majoritaire et des conditions du milieu où se trouvent les colorants anthocyaniques.

Or, à la suite d'importantes recherches menées dans le domaine de la coloration artificielle de la peau, la Demanderesse a maintenant découvert que l'utilisation à titre d'agent de coloration de la peau du chlorure de 4',5,7-trihydroxyflavylium, permettait de conférer immédiatement après l'application sur la peau du produit une coloration artificielle proche du bronzage naturel.

La présente invention a donc pour objet l'utilisation du chlorure d'apigéninidine (ou chlorure de 4', 5, 7-trihydroxyflavylium), obtenu par voie de synthèse ou à partir d'un extrait végétal le contenant ou bien encore à partir d'un extrait végétal enrichi, dans le but de conférer à la peau une coloration artificielle proche du bronzage naturel.

L'utilisation conforme à l'invention permet d'obtenir une coloration artificielle proche du bronzage naturel en un laps de temps court. Ainsi, on obtient une coloration immédiate qui permet une visualisation de l'application et par conséquent une meilleure homogénéité dans l'étalement de la composition sur la peau et donc de la coloration qui en résulte. De plus, la coloration artificielle obtenue sur la peau selon l'invention est extrêmement proche du bronzage naturel.

Au sens de la présente invention, on entendra, par « destinée à la coloration artificielle de la peau », une formulation ayant une affinité particulière pour la peau lui permettant de conférer à cette dernière une coloration durable, non-couvrante (à savoir n'ayant pas tendance à opacifier la peau) et qui ne s'élimine ni à l'eau ni à l'aide d'un solvant, et qui résiste à la fois au frottement et au lavage par une solution contenant des tensioactifs. Une telle coloration durable se distingue donc de la coloration superficielle et momentanée apportée par exemple par un produit de maquillage.

D'autres caractéristiques, aspects et avantages de la présente invention apparaîtront à la lecture de la description détaillée qui va suivre.

Les compositions conformes à la présente invention permettent d'obtenir au bout de 30 minutes après application sur une peau claire à raison de $2mg/cm^2$ un assombrissement caractérisé dans le système de mesure colorimétrique (L*, a*, b*) par un ΔL* allant de -0,5 à - 20. De façon préférentielle, ΔL* variera de - 0,5 à - 15.

Les compositions conformes à la présente invention apportent au bout de 30 minutes après application sur la peau à raison de $2mg/cm^2$ une coloration sur une peau claire définie dans le système de mesure colorimétrique (L*, a*, b*), par un rapport Δa*/Δb* allant de 0,5 à 3 et encore plus particulièrement allant de 0, 8 à 2.

Selon la présente invention, on entend « par peau claire », des peaux non bronzées dont on peut définir les caractéristiques colorimétriques par leur angle ITA tel que défini dans la publication de A. Chardon et al. « Skin Colour Typology and Suntanning Pathways » et présentée au $16^{th}$ IFSCC Congress oct 8-10 1990 de New-York, et in *Int. J. Cosm. Sci.* **13** 191-208 (1991). Les peaux claires telles que définies dans cette classification ont un angle ITA° compris entre 35 et 55.

Dans le système de mesure colorimétrique (L*, a*, b*) :

L* représente la luminance ou clarté, a* représente l'axe rouge-vert (-a*= vert, +a*= rouge) et b* représente l'axe jaune-bleu (-b*=bleu, +b*=jaune). Ainsi, a* et b* expriment la nuance de la peau.
$\Delta L^*$ traduit l'assombrissement de la couleur : plus le $\Delta L^*$ est négatif, plus la couleur s'est assombrie avec :

$$\Delta L^* = L^* \text{ peau non colorée} - L^* \text{ peau colorée}$$

Le rapport $\Delta a^*/\Delta b^*$ traduit l'équilibre rouge/jaune et donc la nuance avec :

$$\Delta a^* = a^* \text{ peau non colorée} - a^* \text{ peau colorée}$$

$$\Delta b^* = b^* \text{ peau non colorée} - b^* \text{ peau colorée}$$

Une forme particulière de l'invention consiste à utiliser le chlorure d'apigéninidine sous forme d'un extrait végétal, aisément préparé par extraction, et isolé, à partir de feuilles de Sorghum caudatum selon les procédés décrits dans les brevets CN 1064284A et CN1035512C ou toutes autres variantes de ces procédés .
Il peut aussi être extrait des tiges, des graines, ou des feuilles de Sorghum Bicolor, des pétales de Gesneria Fulgens, ainsi que des espèces Blechum Procerum et Sorgho en association avec du Colletotrichum Graminicola.
Une forme particulièrement préférée de l'invention est un extrait provenant des feuilles de Sorghum Bicolor obtenu par une extraction hydro-alcoolique en milieu acide à une température d'extraction allant de 30 à 40°C avec un rapport volume de solvant / masse de feuilles de Sorghum Bicolor allant de 10 à 30. Ledit extrait végétal de Sorghum titre environ de 0,05 à 50% en poids de chlorure d'apigéninidine.
Le chlorure d'apigéninidine peut être obtenu par voie de synthèse, et à faible coût, notamment par la méthode bien connue de R. Robinson et D. D. Pratt J. Chem. Soc. 745 (1923). Ladite méthode implique de condenser une orthohydroxybenzaldéhyde ou ses dérivés de substitution sur une acétophénone ou ses dérivés de substitution, pour obtenir, en choisissant les substituants, les composés désirés.
Le schéma de synthèse (i) peut être le suivant :

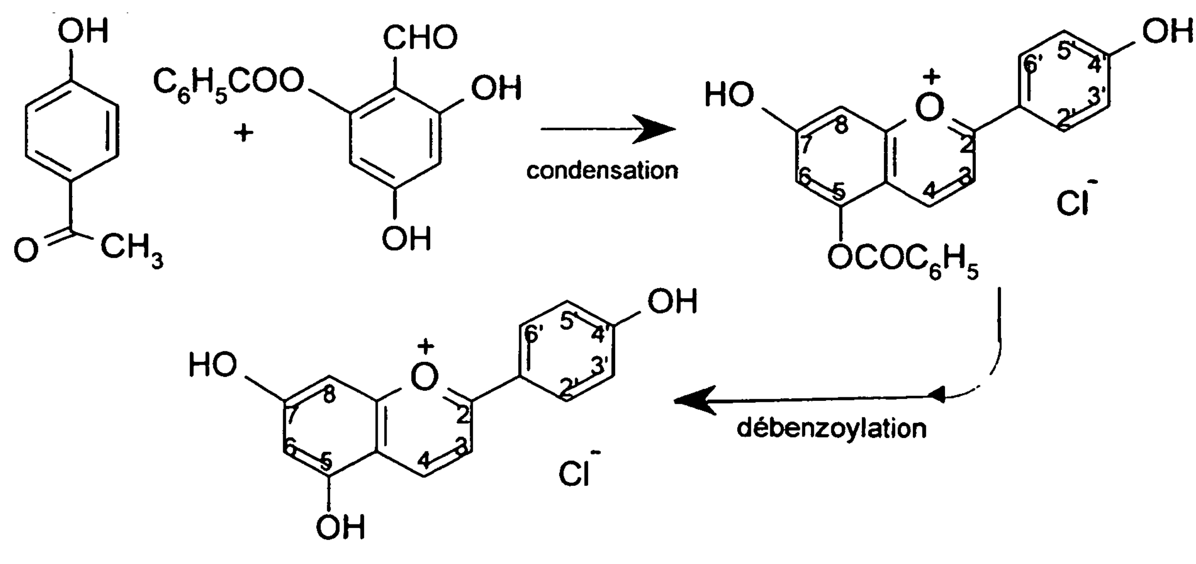


Diverses voies de synthèses, bien connues dans l'art antérieur, conduisent à l'apigéninidine.
Une méthode consiste, par exemple, à préparer, dans une première étape, la triméthylapigéninidine, par condensation du 4,6-diméthoxy-2-hydroxybenzaldéhyde commercial sur la 4-méthoxyacétophénone commerciale à 0°C en milieu éther anhydre, et saturation par HCl anhydre, pour obtenir après filtration un précipité rouge-orangé de triméthylapigéninidine. Dans une seconde étape, à hydrolyser la triméthylapigéninidine obtenue à l'étape précédente en chlorure d'apigéninidine, la réaction s'effectuant en milieu HI et phénol et AgCl en solution dans le méthanol. Une telle

méthode de synthèse est décrite par R. Robinson et A. Robertson dans J. Chem. Soc. 1951 (1926) et 2196 (1927).

Une autre méthode consiste à condenser le 2, 4, 6-trihydroxybenzaldéhyde sur la 4-hydroxyacétophénone à 0°C en milieu solvant anhydre (acétate d'éthyle par exemple), et saturation par HCl anhydre, pour obtenir le chlorure d'apigéninidine. Une telle méthode est décrite par R. Robinson et A. Robertson dans J. Chem. Soc. 1528 (1928).

Une autre méthode de préparation du chlorure d'apigéninidine consiste à réduire une flavone, la naringénine, ou son dérivé triacétylé, par $NaBH_4$, puis à oxyder le produit obtenu par le chloranil (tétrachloro-1,4-benzoquinone). Ladite méthode est décrite par J. G. Sweeny et G. A. Iacobucci dans la revue Tetrahedron **33** 2923-2927 (1977).

La méthode la plus particulièrement préférée, selon la présente invention, consiste à condenser le 2,4-dihydroxy-6-benzoyloxybenzaldéhyde sur la 4-hydroxyacétophénone à 0°C en milieu acétate d'éthyle anhydre, à saturer par HCl anhydre, puis à débenzoyler le produit obtenu par la soude, afin d'obtenir le chlorure d'apigéninidine avec un rendement élevé, suivant le schéma (i) décrit ci-dessus. Ladite méthode est décrite par R. Robinson et J. C. Bell dans J. Chem. Soc. 813 (1934).

La concentration en composé du type sel de flavylium, tel que décrit selon la présente invention, est de préférence comprise entre environ 0,0001 et 10%, et encore plus préférentiellement entre 0,001 et 5% en poids, par rapport au poids total de la composition.

Les compositions conformes à la présente invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones, les $\alpha$-hydroxyacides, les agents répulsifs contre les insectes, les agents anti-mousse, les agents hydratants, les vitamines, les parfums, les conservateurs, les tensioactifs, les charges, les polymères, les propulseurs, les agents alcalinisants ou acidifiants, les colorants, ou tout autre ingrédient habituellement utilisé dans le domaine cosmétique et/ou dermatologique, en particulier pour la fabrication de compositions antisolaires sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine) ; végétales (huile d'amande douce, de macadamia, de pépin de cassis, de jojoba) ; synthétiques comme le perhydrosqualène, les alcools, les acides ou les esters gras (comme le benzoate d'alcools en $C_{12}$-$C_{15}$ vendu sous la dénomination commerciale « Finsolv TN » par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Selon une forme particulièrement préférée, les compositions selon l'invention contiennent au moins 5% en poids par rapport au poids de la composition d'un ou plusieurs solvants polyhydroxylés. Ces derniers peuvent être choisis parmi les glycols et les éthers de glycol comme l'éthylène glycol, le propylène glycol, le butylène glycol, le dipropylène glycol ou le diéthylène glycol. Et plus particulièrement, les compositions selon l'invention contiennent un mélange d'au moins trois solvants polyhydroxylés différents et encore plus particulièrement un mélange constitué de propylène glycol, de butylène glycol, et de dipropylène glycol.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Bien entendu, l'homme de l'art veillera à choisir le ou les éventuels composés complémentaires cités ci-dessus et/ou leurs quantités de manière telle que les propriétés avantageuses attachées intrinsèquement à l'utilisation des composés du type sel de flavylium conformes à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans huile.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. **13**, 238 (1965), FR 2 315 991 et FR 2 416 008).

Des exemples concrets, mais nullement limitatifs, illustrant l'invention, vont maintenant être donnés.

**EXEMPLE 1 :**

On prépare un extrait de Sorghum Bicolor titrant à 20-30% de chlorure d'apigéninidine selon le procédé de préparation suivant :
un extrait provenant des feuilles de Sorghum Bicolor est obtenu par extraction hydro-alcoolique (éthanol 95°) en milieu acide (0.2% HCl) à une température d'extraction de 35°C avec un rapport volume de solvant / masse de feuilles de Sorghum Bicolor de 15. L'extrait végétal de Sorghum est séché à l'étuve 24 h à 40°C et tamisé à 200 µm.
Le rendement de cette extraction est de 22.42% en matière colorante.
Le titre de l'extrait ainsi obtenu est de 21% en poids de chlorure d'apigéninidine.
Cet exemple vise à montrer, dans un premier temps, l'intensité de la coloration obtenue avec un extrait de Sorghum Bicolor conforme à la présente invention ainsi que la rapidité avec laquelle cette coloration se développe par rapport à une composition contenant la DHA à titre d'agent de coloration de la peau.
Cet exemple vise à montrer, dans un deuxième temps, que la coloration obtenue avec un extrait de Sorghum Bicolor conforme à la présente invention est proche de celle du bronzage naturel de la peau.
La Demanderesse a réalisé les compositions suivantes (les quantités sont exprimées en pourcentage de poids par rapport au poids total de la composition) :

Composition A (hors invention):

- Polydiméthyl/méthyl siloxane POE/POP(396/4) (OE/OP 18/18) A 10 % D5 10g
- Cyclopentadiméthylsiloxane 12.5g
- Mélange tocophérols naturels/huile de soja 0.1g
- Dihydroxyacétone (DHA) 4g
- Chlorure de sodium 2g
- Propylène glycol 23g
- Butylène glycol 5g
- Dipropylène glycol 10g
- Eau déminéralisée 32.649 g
- Citrate trisodique 0.542g
- Acide citrique 0.209g

Composition B (invention)

- Polydiméthyl/méthyl siloxane POE/POP(396/4) (OE/OP 18/18) A 10 % D5 10g
- Cyclopentadiméthylsiloxane 12.5g
- Mélange tocophérols naturels/huile de soja 0.1g
- Eau déminéralisée 36.159g
- Chlorure de sodium 2g
- Propylène glycol 23g
- Butylène glycol 5g
- Dipropylène glycol 10g
- Extrait de Sorghum Bicolor tel que préparé ci-dessus 0.5g
- Citrate trisodique 0.535g
- Acide citrique 0.206g

**Protocole d'évaluation :**

Les compositions A et B ont été appliquées à raison de 2 $mg/cm^2$ sur une zone de 7 x 4,5 $cm^2$ délimitée sur le dos de 6 volontaires dont la couleur de peau caractérisée par l'angle ITA est compris entre 35 et 55.).
Les cinq séries de mesures colorimétriques suivantes ont été effectuées à l'aide d'un colorimètre Minolta CR-300 :

- 1°) avant application de la composition,
- 2°) 30 minutes après l'application,
- 3°) 2 heures après application.
- 4°) 4 heures après application.
- 5°) 5 heures après application

Les résultats sont exprimés dans le système (L*, a*, b*) dans lequel L* représente la luminance, a* représente l'axe rouge-vert (-a* = vert, +a* = rouge) et b* représente l'axe jaune-bleu (-b* =bleu, +b* =jaune). Ainsi, a* et b* expriment la nuance de la peau.
Pour l'évaluation de l'intensité de la coloration, on s'intéresse à ΔL* qui traduit l'assombrissement de la couleur : plus ΔL* est négatif, plus la couleur s'est assombrie avec :

$$\Delta L^* = L^* \text{ peau non colorée} - L^* \text{ peau colorée}$$

Pour la nuance de la coloration obtenue, on s'intéresse au rapport Δa*/Δb* qui traduit l'équilibre rouge/jaune et donc la nuance avec :

$$\Delta a^* = a^* \text{ peau non colorée} - a^* \text{ peau colorée}$$

$$\Delta b^* = b^* \text{ peau non colorée} - b^* \text{ peau colorée}$$

Les résultats obtenus sont rassemblés dans le tableau (I) suivant :

Tableau (I):

| | **Composition A (comparative) ΔL*** | **Composition B (invention) ΔL*** | **Composition B (invention) Δa*/Δb*** |
|---|---|---|---|
| 30 minutes | - 0,4 | - 8,2 | 1,6 |
| 2 heures | -1,1 | - 7 | 1,4 |
| 4 heures | - 2,5 | - 6,7 | 1,6 |
| 5 heures | - 2,6 | - 6,9 | 1,7 |

On constate ainsi que 30 minutes après l'application, la composition A, qui contient à titre d'agent de coloration de la peau, la DHA, n'a conféré qu'une très faible coloration à la peau, puisque la DHA n'a pas encore eu le temps d'agir (ΔL* = - 0,4). En revanche, la composition B selon l'invention a déjà conféré à la peau une coloration significative (ΔL* = - 8,2).
La composition A contenant la DHA ne permet pas d'obtenir au bout de 30 minutes un assombrissement comparable à celui de la composition B qui présente de plus une nuance de coloration constante pendant 5 heures.
La composition B conduit à une coloration sur la peau proche du bronzage naturel et constante dans le temps.

**Revendications**

1. Utilisation du chlorure de 4', 5, 7-trihydroxyflavylium obtenu par voie de synthèse ou à partir d'un extrait végétal le contenant ou bien encore à partir d'un extrait végétal enrichi pour conférer à la peau une coloration artificielle proche du bronzage naturel dans une composition cosmétique.

2. Utilisation selon la revendication 1, **caractérisée par le fait qu'**il s'agit du chlorure de 4', 5, 7-trihydroxyflavylium, sous forme pure.

3. Utilisation selon l'une quelconque des revendications 1 à 2, **caractérisée par le fait qu'**il s'agit du chlorure de 4', 5, 7-trihydroxytlavylium, sous forme d'extrait végétal.

4. Utilisation selon la revendication 3, **caractérisée par le fait que** l'extrait végétal est un extrait végétal obtenu à partir de feuilles de Sorghum caudatum, ; de tiges, de graines ou de feuilles de Sorghum Bicolor ; des pétales de Gesneria Fulgens, ainsi que des espèces Blechum Procerum et Sorgho en association avec du Colletotrichum Graminicola.

5. Utilisation selon la revendication 4, **caractérisée par le fait que** l'extrait végétal est un extrait de Sorghum Bicolor susceptible d'être obtenu par une extraction hydro-alcoolique acide à une température d'extraction allant de 30 à 40°C avec un rapport volume de solvant 1 masse de feuilles de Sorghum Bicolor allant de 10 à 30.

6. Utilisation selon la revendication 5, **caractérisée par le fait que** l'extrait de Sorghum Bicolor titre de 0,05 à 50% en poids de chlorure de 4', 5, 7-trihydroxyflavylium

7. Utilisation selon l'une quelconque des revendications 1 à 8 où la concentration en chlorure de 4', 5, 7-trihydroxyflavylium varie de comprise entre environ 0,0001 et 10% en poids par rapport au poids total de la composition.

8. Utilisation selon la revendication 7, où la concentration en chlorure de 4', 5, 7-trihydroxyflavylium varie de 0,001 et 5% en poids, par rapport au poids total de la composition.

9. Utilisation selon l'une quelconque des revendications 1 à 8, où la composition contient au moins 5% en poids par rapport au poids de la composition d'un ou plusieurs solvants polyhydroxylés.

10. Utilisation selon la revendication 9, où les solvants polyhydroxytés sont choisis parmi les glycols et les éthers de glycol.

11. Utilisation selon la revendication 9 ou 10, où les solvants polyhydroxylés sont choisis parmi l'éthylène glycol, le propylène glycol, le butylène glycol, le dipropylène glycol ou le diéthylène glycol et leurs mélanges.

12. Utilisation selon l'une quelconque des revendications 9 à 11, où la composition contient un mélange de trois solvants polyhydroxylés différents.

13. Utilisation selon la revendication 12, où la composition contient un mélange constitué de propylène glycol, de butylène glycol, et de dipropylène glycol.

**Claims**

1. Use of 4',5,7-trihydroxyflavyliiam chloride, obtained by the synthetic route or from a plant extract comprising it or alternatively from an enriched plant extract, in a cosmetic composition for conferring, on the skin, an artificial colouring similar to natural tanning.

2. Use according to Claim 1, **characterized in that** it relates to 4',5,7-trihydroxyflavylium chloride in the pure form.

3. Use according to either one of Claims 1 and 2, **characterized in that** it relates to 4',5,7-trihydroxyflavylium chloride in the form of a plant extract.

4. Use according to Claim 3, **characterized in that** the plant extract is a plant extract obtained from leaves of Sorghum caudatum; from stems, seeds or leaves of Sorghum bicolor; from the petals of Gesneria fulgens, and from the species Blechnum procerum and sorghum in combination with Colletotrichum graminicola.

5. Use according to Claim 4, **characterized in that** the plant extract is an extract of Sorghum bicolor capable of being obtained by an acidic water/alcohol extraction at an extraction temperature ranging from 30 to 40°C with a volume of solvent/weight of Sorghum bicolor leaves ratio ranging from 10 to 30.

6. Use according to Claim 5, **characterized in that** the Sorghum bicolor extract assays from 0.05. to 50% by weight of 4',5,7-trihydroxyflavylium chloride.

7. Use according to any one of Claims 1 to 6, where the concentration of 4',5,7-trihydroxyflavylium chloride is between approximately 0.0401, and 10% by weight with respect to the total weight of the composition.

8. Use according to Claim 7, where the concentration of 4',5,7-trihydroxyflavylium chloride is between 0.001 and 5% by weight with respect to the total weight of the composition.

9. Use according to any one of Claims 1 to 8, where the composition comprises at least 5% by weight, with respect to the weight of the composition, of one or more polyhydroxylated solvents.

10. Use according to Claim 9, where the polyhydroxylated solvents are chosen from glycols and glycol ethers.

11. Use according to Claim 9 or 10, where the polyhydroxylated solvents are chosen from ethylene glycol, propylene glycol, butylene glycol, dipropylene glycol or diethylene glycol and their mixtures.

12. Use according to any one of Claims 9 to 11, where the composition comprises a mixture of three different polyhydroxylated solvents.

13. Use according to Claim 12, where the composition comprises a mixture composed of propylene glycol, of butylene glycol and of dipropylene glycol.

**Patentansprüche**

1. Verwendung von 4',5,7-Trihydroxyflavyliumchlorid, das auf synthetischem Weg oder aus einem diese Verbindung enthaltenden Pflanzenextrakt oder aus einem angereicherten Pflanzenextrakt erhalten ist, zur Erzielung einer künstlichen Färbung der Haut, die der natürlichen Bräune nahe kommt, in einer kosmetischen Zusammensetzung.

2. Verwendung nach Anspruch 1, **dadurch gekennzeichnet, dass** es sich um 4',5,7-Trihydroxyflavyliumchlorid in reiner Form handelt.

3. Verwendung nach Anspruch 1 oder 2, **dadurch gekennzeichnet, dass** es sich um 4', 5, 7-Trihydroxyflavyliumchlorid in Form eines Pflanzenextraktes handelt.

4. Verwendung nach Anspruch 3, **dadurch gekennzeichnet, dass** der Pflanzenextrakt ein Pflanzenextrakt ist, der erhalten ist aus Blättern von Sorghum caudatum; Stengeln, Samen oder Blättern von Sorghum bicolor; Blütenblättern von Gesneria fulgens sowie den Species Blechum procerum und Sorgho in Kombination mit Colletotrichum graminicola.

5. Verwendung nach Anspruch 4, **dadurch gekennzeichnet, dass** der Pflanzenextrakt ein Extrakt von Sorghum bicolor ist, der erhältlich ist durch wässerig-alkoholische saure Extraktion bei einer Extraktionstemperatur von 30 bis 40 °C und einem Verhältnis Volumen des Lösungsmittels/Masse der Blätter von Sorghum bicolor von 10 bis 30.

6. Verwendung nach Anspruch 5, **dadurch gekennzeichnet, dass** der Extrakt von Sorghum bicolor einen Gehalt an 4',5,7-Trihydroxyflavyliumchlorid von 0,05 bis 50 Gew.-% aufweist.

7. Verwendung nach einem der Ansprüche 1 bis 6, wobei die Konzentration an 4',5,7-Trihydroxyflavyliumchlorid im Bereich von 0,0001 bis 10 Gew.-%, bezogen auf das Gesamtgewicht der Zusammensetzung, variiert.

8. Verwendung nach Anspruch 7, wobei die Konzentration an 4',5,7-Trihydroxyflavyliumchlorid im Bereich von 0,001 bis 5 Gew.-%, bezogen auf das Gesamtgewicht der Zusammensetzung, variiert.

9. Verwendung nach einem der Ansprüche 1 bis 8, wobei die Zusammensetzung mindestens 5 Gew.-% eines oder mehrerer Polyhydroxy-Lösungsmittel enthält, bezogen auf das Gewicht der Zusammensetzung.

10. Verwendung nach Anspruch 9, wobei die Polyhydroxy-Lösungsmittel unter Glykolen und Glykolethern ausgewählt sind.

11. Verwendung nach Anspruch 9 oder 10, wobei die Polyhydroxy-Lösungsmittel ausgewählt sind unter Ethylenglykol, Propylenglykol, Butylenglykol, Dipropylenglykol oder Diethylenglykol sowie Gemischen dieser Verbindungen.

12. Verwendung nach einem der Ansprüche 9 bis 11, wobei die Zusammensetzung ein Gemisch von 3 verschiedenen

Polyhydroxy-Lösungsmitteln enthält.

**13.** Verwendung nach Anspruch 12, wobei die Zusammensetzung ein Gemisch enthält, das aus Propylenglykol, Butylenglykol und Dipropylenglykol besteht.